Europäisches Patentamt

European Patent Office

Office européen des brevets

(11) Publication number: 0 230 672
A2

(12) **EUROPEAN PATENT APPLICATION**

(21) Application number: 86118156.8

(22) Date of filing: 30.12.86

(51) Int. Cl.³: **A 61 F 2/12**

(30) Priority: 30.12.85 US 814320

(43) Date of publication of application:
05.08.87 Bulletin 87/32

(84) Designated Contracting States:
DE FR GB

(71) Applicant: AMERICAN HOSPITAL SUPPLY CORPORATION
One Baxter Parkway
Deerfield Illinois 60015(US)

(72) Inventor: Nashef, Aws Salim
2824 Corvo Place
Costa Mesa California 92626(US)

(72) Inventor: Campbell, Todd Duncan
1333 Turquoise drive
Corona California 91720(US)

(74) Representative: Patentanwälte Grünecker, Kinkeldey, Stockmair & Partner
Maximilianstrasse 58
D-8000 München 22(DE)

(54) Method for implantation of mammary prosthesis.

(57) A method for implantation of a mammary prosthesis involves interposing a barrier layer of soft biological tissue at the interface of the prosthesis and the surrounding host tissue. This barrier layer reduces the formation of fibrous connective tissue capsules and subsequent hardening of the scar tissue that may occur following implantation of mammary prosthesis.

FIG. 1.

64-180A
)A:580

METHOD FOR IMPLANTATION OF
MAMMARY PROSTHESIS

## BACKGROUND OF THE INVENTION

The present invention relates to a method for implantation of a mammary prosthesis. More particularly, the invention concerns such a method, wherein the formation and contraction of fibrous tissue around the prosthesis is minimized.

Silicone-filled breast implants are widely used in cosmetic and reconstructive surgery of the breasts. A problem associated with the healing process following implantation of the mammary prosthesis often arises in the tissue surrounding the prosthesis. Implantation of the mammary prosthesis into the living tissue of the host gives rise to a well-defined host response that may lead to the formation of a fibrous connective tissue capsule surrounding the prosthesis. The implant itself does not harden, but the scar tissue that forms the capsule may contract, resulting in undesirable hardening of the tissue around the implant. This contraction of tissue may also compress the silicone implant, resulting in an aesthetically undesirable spherical shape. Certain individuals seem to be more prone to the above-described problems than others. Replacement of the implants with new silicone prostheses results in the same hardened capsule formation in these individuals. It has been estimated that 25 to 30 percent of women having silicone breast implants experience problems with contraction of fibrous capsules.

Transmission electron microscopy studies indicate that myofibroblasts (MFB) probably play a major role in the contraction of tissues around silicone breast implants. The appearance of MFB's seems to occur early in the wound healing process, and the MFB's have a well-defined life span. MFB formation is triggered by a

2

stimulus, causing contraction around the implant. As the wound ages, the quantity of MFB diminishes and the surrounding capsule is comprised mostly of dense collagen.

Various attempts to minimize the formation and contraction of scar tissue have met with only limited success. Conventional therapy involves massage of the mammary prosthesis to stretch the scar as it forms. The massage procedure reduces the overall degree of contraction. Other therapies include topical use of slow release steroids, e.g., glucocorticoids, and oral administration of vitamin E. These remedies can be very inconvenient to the patient and can offer only a response to rather than a prevention of, the formation of capsular contraction. Capsular contraction of scar tissue thus remains a critical drawback in the use of silicone implants.

## SUMMARY OF THE INVENTION

In accordance with the present invention a method for implantation of a mammary prosthesis involves interposing a barrier layer of soft biological tissue at the interface of said mammary prosthesis and the surrounding host tissue, said layer of biological tissue having overall dimensions sufficient to substantially cover the outer surface of the prosthesis and separate it from the surrounding tissue.

## BRIEF DESCRIPTION OF THE DRAWINGS

Fig. 1 is a cross-sectional view of a mammary prosthesis which has been implanted according to the method of the present invention.

Fig. 2 is a cross-sectional view similar to that of Fig. 1, showing an alternative embodiment of the present invention.

Fig. 3 is a cross-sectional view similar to that of Figs. 1 and 2, showing the soft biological material in the form of a pouch with an opening in the posterior sheath for insertion of the mammary prosthesis.

Fig. 4 is a rear view of a mammary prosthesis enclosed within a sheath of soft biological material.

## DETAILED DESCRIPTION OF THE INVENTION

In accordance with the present invention, a soft biological material is interposed between a mammary prosthesis and the surrounding host tissue. By way of example, a mammary prosthesis may be sheathed with a sheath of bioprosthetic material during surgical implantation of the prosthesis. The bioprosthetic material advantageously inhibits the formation of a fibrous connective tissue capsule which, in turn, reduces capsular contraction. The material also protects surrounding tissue from the deleterious effects caused by silicone and silicone gel that may, on rare occasions, fragment or exude from silicone implants.

The bioprosthetic material may comprise a single sack-like sheath of biological tissue into which the prosthesis is inserted prior to implantation. Preferably, the bioprosthetic material comprises two separate sheaths, which may or may not be sutured together, as described below.

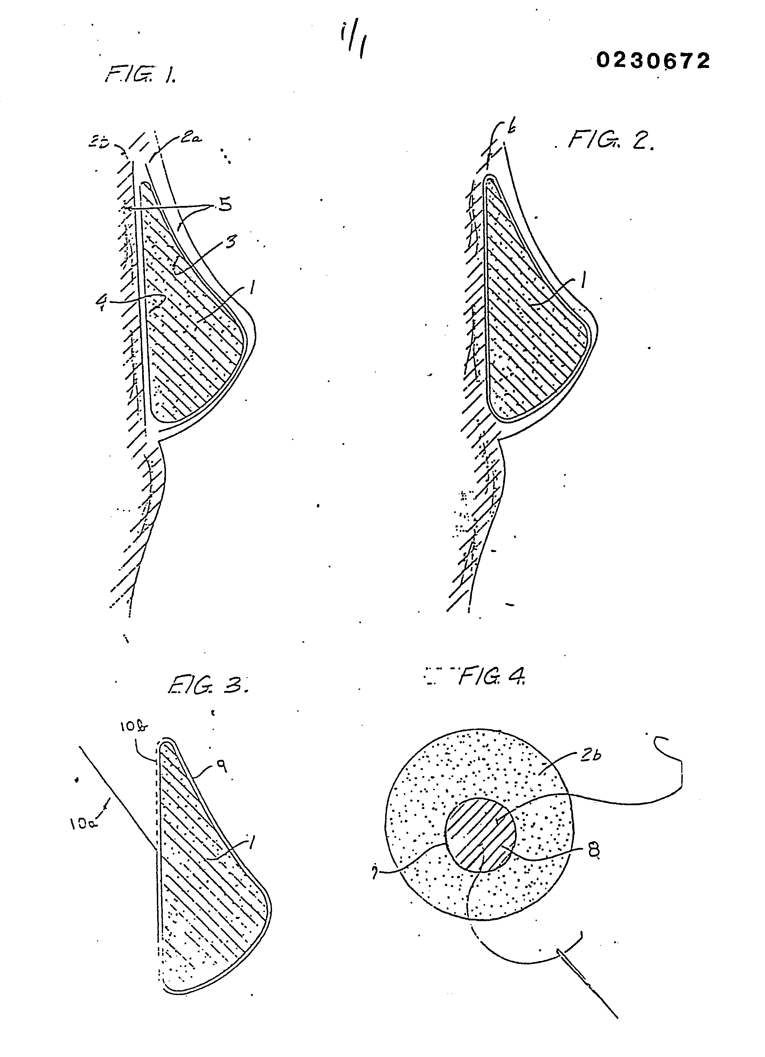

With reference to the drawings, Fig. 1 illustrates the surgical placement of the bioprosthetic material around the mammary prosthesis. In the illustrative embodiment, the bioprosthetic material includes two sheaths, 2a and 2b, that may be applied to the anterior surface 3 and the posterior surface 4, respectively, of the mammary prosthesis 1 to form an interface and barrier between the prosthesis and the surrounding host tissue 5. Alternatively, anterior and posterior sheaths may be

4

sutured together to form a single sheath 6 surrounding the prosthesis 1 as shown in Fig. 2. Preferably, the bioprosthetic tissue incorporates an opening 7 in the posterior sheath 2b to accommodate a fixation area for attachment of the prosthesis to the chest wall. Such a fixation area may, for example, be a suturing disc 8 of Dacron® or other material as shown in Fig. 4.

In accordance with the present invention, the bioprosthetic material is in sheet form and is inserted between the implanted mammary prosthesis and the surrounding host tissues during surgical implantation of the mammary prosthesis. The bioprosthetic tissue may be free-floating and held in place by friction with the mammary prosthesis and with the surrounding host tissue. Alternatively, it may be secured to the mammary prosthesis by any suitable means such as sutures.

In an alternative embodiment of the present invention, the anterior and posterior sheaths of bioprosthetic material may be surgically implanted prior to implantation of the mammary prosthesis. The sheaths are sutured together to form a unitary sheath 9 that defines a sack whose internal dimensions are sufficient to receive the mammary prosthesis (Fig. 3). A portion of the sack remains unsutured to form a flap 10a and an opening 10b through which the mammary prosthesis is inserted. After insertion of the prosthesis 1 into sheath 9, flap 10a can be folded back to meet the anterior portion of the sheath thus closing opening 10b, and the unsutured portions are sutured together to form the unitary protective sheath.

The shape and dimensions of the bioprosthetic material will vary depending upon the shape and surface area of the mammary prosthesis. Generally, the posterior sheath is disc-shaped and the anterior sheath conforms to the contours of the mammary prosthesis. In a preferred embodiment of the invention, the bioprosthetic material has dimensions sufficient to cover the entire surface of the mammary prosthesis.

The bioprosthetic material is made of soft biological tissue, such as naturally occurring soft biological tissue derived from animal sources. Suitable animal sources can include but are not limited to bovine, porcine, horse, sheep, kangaroo, rabbit or human cadavers. Naturally occuring biological tissue in accordance with the present invention includes, but is not limited to, epithelial and fibrous connective tissue such as pericardial tissue, dura mater, fascia lata, amnion, tendon, ligament, cartilage, and the like. The epithelial tissues, such as dura mater, amnion, facia lata, and pericardium generally comprise two layers: a rough fibrous layer and a relatively smooth membranous layer. In accordance with a preferred embodiment of the present invention, the rough, fibrous layer of the tissue is placed against the mammary prosthesis to provide better anchoring to the prosthesis, while the smooth, membranous layer is directed toward the surrounding tissue and provides a more lubricious surface. In accordance with the preferred embodiment of the present invention, pericardial tissue which has been tanned with glutaraldehyde, as described below, is employed as the interpositional bioprosthetic material.

Alternatively, the soft biological tissue may comprise collagen or reconstituted collagen substitutes including but not limited to collagen-fabric films, collagen membranes, reconstituted collagen on synthetic Dacron® mesh, tanned collagen sponge grafts and the like. These materials have been found to elicit minimal host reaction and fibrous reaction and thus inhibit the

6

formation and subsequent contraction of fibrous capsules around the prosthesis.

In preparing the bioprosthetic material in accordance with a preferred embodiment of the invention, naturally occurring biological tissue is removed from its host, defatted if necessary and processed by one of several well-known procedures used to prepare the tissue for implantation into humans. The tissue is fixed (tanned) conventionally in a solution containing from about 0.2 percent to about 0.8 percent (w/v), preferably about 0.6 percent (w/v) glutaraldehyde in either phosphate-buffered saline solutions, or phosphate-free buffers as described in the copending U.S. patent application Serial No. 445,345 filed on November 29, 1982, the disclosure of which is incorporated herein by reference. The tissue is advantageously contacted with the tanning (fixing) agent for a time sufficient to cross-link proteins in the tissue to a degree sufficient to stabilize the tissue and to render it substantially non-antigenic. The non-antigenic tissue can be derived from many different animal species, as described above, and implanted in a human without causing an adverse immunological reaction. The tissue handling conditions, as conventionally known, are not considered part of the present invention unless otherwise stated. Likewise, tissue may be sterilized conventionally, by such means as radiation or exposure to ethylene oxide, or by immersion in a solution comprising glutaraldehyde or about 4-5 percent formaldehyde.

In accordance with a preferred embodiment of the present invention, the natural biological tissue is treated prior to implantation to render it substantially resistant to calcification in the body. This treatment advantageously maintains the biological tissue in a more flexible state than calcified tissue, allowing the tissue to conform better to the surface of the mammary prosthesis. Calcification mitigation treatments of implantable biological tissue are known and are described, for example, in copending U.S. patent applications serial numbers 445,345 filed November 29, 1982; and 441,023 filed November 12, 1982; and in U.S. Patent Nos. 4,323,358 and 4,481,009.

Advantageously, the tissue is immersed in a solution which serves to both sterilize the tissue and to mitigate calcification. Such a solution may be a buffered surfactant/formaldehyde/ethanol solution.

As used herein, "mammary prosthesis" is meant to refer not only to silicone implants, but also to mammary prostheses made of other materials which elicit adverse host responses such as fibrous capsule formation after implantation. The prostheses may be used to augment or to reconstruct the breasts.

The following example is provided to illustrate one embodiment of the invention, and is not to be construed as limiting.

## EXAMPLE I

Extracted bovine pericardial tissue was thoroughly rinsed and shipped in an isotonic (285 15 milliosmols) solution containing 0.54 grams/liter of the sodium salt of HEPES and 0.885 weight percent sodium chloride at pH 7.3 at about 4°C. The tissue was fixed with 0.625 weight percent glutaraldehyde in an isotonic solution containing 5.39 grams/liter of the sodium salt of HEPES, 0.440 weight percent sodium chloride, and 2.6 grams/liter of $MgCl_2 \cdot 6H_2O$ at room temperature for about 24 hours or longer.

8

Disc-shaped pieces conforming to the outer surfaces of mammary prostheses were cut from this fixed tissue. The tissue was then further sterilized in a 4% formaldehyde/22.5% ethanol/1.2% Tween - 80™ solution buffered with HEPES, pH 7.4 at 37°C for 9 hours. The tissue was rinsed in sterile saline to remove residual glutaraldehyde at a time immediately prior to implantation.

The sterilized pericardial tissue discs were then placed over silicone mammary prostheses, and the thus-sheathed prostheses were then implanted using standard surgical techniques. The patients included several women who had experienced problems with recurring capsule formation and hardening of scar tissue following previous implantations of silicone mammary prostheses lacking the barrier layer, i.e., sheath, of biological tissue. Four months after implantation of the sheathed prostheses, no hardening of scar tissue was detected in any of the patients, indicating that capsule formation was drastically minimized. The implanted tissue covering the prostheses was thus found to be biocompatible and effective in reducing the formation of fibrous connective tissue capsules around the prosthesis.

The present invention has been described in detail and with specific reference to its preferred embodiments; however, it will be understood by those skilled in the art that modifications can be made thereto without departing from the spirit and scope thereof.

## CLAIMS

What Is Claimed Is:

1. The use of soft biological tissue fixed with a fixing agent as a barrier layer having dimensions sufficient to substantially cover the outer surface of a mammary prosthesis, wherein said barrier layer is interposed at the interface of said mammary prosthesis and the surrounding host tissue, thereby inhibiting the formation of a fibrous connective tissue capsule around said prosthesis.

2. The use of claim 1, wherein the barrier layer comprises naturally occurring soft biological tissue fixed with a fixing agent.

3. The use of claim 2, wherein the biological tissue is epithelial tissue or fibrous connective tissue.

4. The use of claim 3, wherein the biological tissue is chosen from pericardial tissue, fascia lata, dura mater, amnion, tendon, ligament or cartilage.

5. The use of claim 4, wherein the biological tissue is pericardial tissue.

6. The use of claims 2, 3, 4 or 5, wherein said biological tissue is taken from an animal source selected from bovine, porcine, horse, sheep, kangaroo, or rabbit sources, or from a human cadaver.

7. The use of claim 1 or 2, wherein the fixing agent is glutaraldehyde.

8. The use of claim 1 or 2, wherein the biological tissue is treated to render it substantially resistant to calcification.

9. The use of claim 1, wherein said biological tissue comprises collagen or a reconstituted collagen substitute chosen from collagen-fabric films, reconstituted collagen on a synthetic mesh, collagen sponge grafts, or collagen membranes.

10. A sheath for interposing between an implanted mammary prosthesis and the surrounding tissue of a host, which comprises a barrier layer of soft biological tissue, fixed with a fixing agent, having a means therein for attachment of said prosthesis to the host, and having overall dimensions sufficient to substantially cover the prosthesis, thereby inhibiting the formation of a fibrous connective tissue capsule around said prosthesis.

11. The sheath of claim 10, wherein said barrier layer comprises naturally occurring biological tissue fixed with a fixing agent.

12. The sheath of claim 11, wherein said biological tissue is epithelial tissue or fibrous connective tissue.

13. The sheath of claim 12, wherein said biological tissue is chosen from pericardial tissue, _fascia_ _lata_, _dura_ _mater_, amnion, tendon, ligament, or cartilage.

14. The sheath of claim 13, wherein said biological tissue is pericardial tissue.

15. The sheath of claims 10, 11, 12, 13 or 14, wherein said biological tissue is taken from an animal source selected from bovine, porcine, horse, sheep, kangaroo, or rabbit sources, or from a human cadaver.

16. The sheath of claim 10 or 11, wherein the fixing agent is glutaraldehyde.

17. The sheath of claim 10, wherein said biological tissue is treated to render it substantially resistant to calcification.

18. The sheath of claim 10, wherein said biological tissue comprises collagen or a reconstituted collagen substitute chosen from collagen-fabric films, reconstituted collagen on a synthetic mesh, collagen sponge grafts, or collagen membranes.

19. The sheath of claim 10, wherein the attachment means is an aperture through said soft biological tissue covering the posterior surface of the prosthesis.

20. The sheath of claim 10, wherein said biological tissue covering the anterior surface of the prosthesis is contoured to conform to the shape of the anterior surface of the prosthesis.

21. The sheath of claim 10, which comprises two or more separate sheets of said biological tissue.

22. The sheath of claim 21, wherein one sheet of said biological tissue is interposed between the anterior surface of the prosthesis and the host tissue, and a second sheet of said biological tissue is interposed between the posterior surface of the prosthesis and the host tissue.

23. The sheath of claim 21, wherein said sheets of biological tissue are sutured together to form a single sheath.

24. The sheath of claim 10, which comprises a single sheet of biological tissue.

FIG. 1.

FIG. 2.

FIG. 3.

FIG. 4.

0230672